# EUROPEAN PATENT SPECIFICATION

(11) **EP 2 145 635 B1**
(45) Date of publication and mention of the grant of the patent: **09.12.2015**
(21) Application number: 08750425.4
(22) Date of filing: 31.03.2008
(51) Int. Cl.: A61L 27/22, A61L 27/58, A61L 27/38, A61L 27/60

(54) **METHOD FOR PREPARING THREE-DIMENSIONAL STRUCTURES FOR TISSUE ENGINEERING**
VERFAHREN ZUR HERSTELLUNG DREIDIMENSIONALER STRUKTUREN FÜR GEWEBEZÜCHTUNG
PROCÉDÉ D'OBTENTION DE STRUCTURES TRIDIMENSIONNELLES DESTINÉES AU GÉNIE TISSULAIRE

(30) Priority: 29.03.2007 ES 200700835
(43) Date of publication of application: 20.01.2010
(73) Proprietor: Centro Comunitario De Sangre Y Tejidos De Asturias, 33006 Oviedo (ES); Centro de Investigaciones Energéticas, Medioambientales y Tecnológicas, 28040 Madrid (ES)
(72) Inventor: MEANA INFIESTA, Alvaro, E-33006 Oviedo (ES); GARCIA PEREZ, Eva, E-33006 Oviedo (ES); GARCIA DIAZ, Veronica, E-33006 Oviedo (ES); JORCANO NOVAL, Jose, Luis, E-28040 Madrid (ES); DEL RIO NECHAEVSKY, Marcela, E-28040 Madrid (ES); LARCHER LAGUZZI, Fernando, E-28040 Madrid (ES); DUARTE GONZALEZ, Blanca, E-28040 Madrid (ES); HOLGUIN FERNANDEZ, Almudena, E-28040 Madrid (ES)
(74) Representative: Carpintero Lopez, Francisco
(86) International application number: PCT/ES2008/000191
(87) International publication number: WO 2008/119855

(56) References cited:
- EP-A1- 1 535 633
- WO-A1-2004/108810
- WO-A1-2006/099137
- DE-A1- 3 517 456
- US-A1- 2002 059 001
- US-A1- 2002 059 001
- YOUNG S. ET AL.: 'Gelatin as a delivery vehicle for the controlled release of bioactive molecules' JOURNAL OF CONTROLLED RELEASE vol. 109, no. 1-3, 2005, pages 256 - 274, XP005196220
- MA L. ET AL.: 'Collagen/chitosan porous scaffolds with improved biostability for skin tissue engineering' BIOMATERIALS vol. 24, no. 26, 01 November 2003, pages 4833 - 4841, XP004460200

## Description

### Field of the Invention

The present invention belongs to the field of tissue engineering and specifically relates to a method for obtaining three-dimensional structures for tissue engineering and to the structures obtained by said method. The invention also relates to an ex vivo method for regenerating a tissue using the three-dimensional structures of the invention and to the use of the structures thus treated for their transplant to the area to be regenerated of the patient.

### Background of the Invention

Obtaining a new tissue or organ which can substitute the damaged one is one of the objectives of regenerative medicine. To that end, Tissue Engineering techniques have to be used, the basis of which is the reconstruction of the damaged tissue or organ from small healthy tissue remains (Sipe J et al., Ann N. Y. Acad. Sci. 961: xiii-xiv, 2002). The possibility of reconstructing a damaged tissue or organ from cells of other tissues of the organism or even from multipotent embryonic cells has been currently added to this technology.

Cells are an indispensable element in the production of new tissues. The cells form the living component of tissues and are those performing the biological functions characteristic thereof (for example the production of albumin by hepatocytes, the filtration by glomerular cells etc). Cell culture technology allows, from a small number of cells present in a healthy tissue fragment, increasing their number in logarithmic proportions without these cells losing their characteristics. In some cases it also allows transdifferentiating cells (for example from fat cells to bone cells) or even directing undifferentiated cells towards cells of a certain tissue (from embryonic cells towards insulin-producing cells). However, despite all this developed technology, tissues cannot be manufactured with isolated cells, since in such tissues the cells are distributed in a three-dimensional structure and this spatial distribution is critical for the development of the function of the organ or tissue. This three-dimensional structure is provided by the extracellular matrix of the tissues. This matrix is not only a structure for supporting the cells, but rather it has adhesion motifs so that the cells are conveniently fixed. The extracellular matrix is coordinately produced and destroyed by the cells composing the organ or tissue themselves. For this reason, in most tissue engineering models, it is necessary to provide the previously cultured cells with a structure or support (scaffolds) so that once they are introduced therein and placed in three dimensions, the cells behave as the most physiological manner possible (Godbey WT and Atala A. Ann N. Y. Acad Sci. 961: 10-26, 2002).

Unfortunately there is not enough technology to produce extracellular matrices similar to those of adult tissues. The search for these three-dimensional structures, supports, scaffolds is one of the most important fields of research in the Tissue Engineering field (Griffith L, Ann N. Y. Acad Sci, 961: 83-95, 2002).

The scaffold must not only provide a three-dimensional structure, it is necessary for the cells to be capable of adhering thereto. The base product of the scaffold must not contain substances which are toxic for the cells. It also needs other characteristics such as mechanical strength (variable according to the organ or tissue to be reconstructed) or degradation capacity. The ideal scaffold must degrade and allow being substituted with the normal extracellular matrix of the corresponding tissue. The scaffold must have a structure allowing the cells to enter inside it, therefore the most of these structures are manufactured from porous materials. Multiple substances have been used as scaffolds in Tissue Engineering, this gives an idea of the complexity of the needs that an ideal scaffold must have.

Some biopolymers have been widely used as scaffold. Poly(alpha-hydroxyacids) (Kulkarny et al., Arch Surg, 93: 839-843, 1993) such as polyglycolic acid (PGA) or poly-L-lactic acid (PLLA) or combinations of both substances (PLGA) stand out among them.

Other substances of biological origin used in the development of scaffolds are hyaluronates (Itay S et al., Clin Orthop, 220: 234-300, 1987), seashell chitin derivatives (chitosan) (Prasitsilp et al., J Mat Sci: Mat in Med, 11: 773-778, 2000) or alginates (Fragonas et al., Biomaterials 21: 795-801, 2000).

Another possible source of scaffold are the compounds that are the basis of the extracellular matrix in mammals, the clearest example is the use of type I collagen gels to develop Tissue Engineering models (Maraguchi et al., Plast Reconstr Surg, 93: 537-544, 1994). Another substance which is highly used as scaffold is the fibrinogen present in plasma (Gurevitch O. et al., Tissue Engineering 8: 661-672, 2002, Meana et al., Burns 621-630, 1998).

A widely used biological product in therapeutics is albumin. Human albumin or albumin from another mammal species is obtained from plasma. Albumin is normally used in venous infusion, although it has also been used as biological glue, mixing it with a cross-linking product comprising an aldehyde, generally glutaraldehyde. This mixture used in therapeutics produces a tissue adhesive used in surgery and described in application WO 2005/00925. A model in the form of a three-dimensional structure based on albumin-glutaraldehyde is also described, which could be reinforced by means of the inclusion of fibers and/or other components and which could be implanted in the organism (WO 00/70018). Other uses of this model described in the state of the art are the use as microbeads for the gradual delivery of drugs (US 4349530). However, the use of this structure as scaffold or support for Tissue Engineering techniques has not been possible since it is a solid structure, without pores inside it so as the cells can enter. Furthermore, glutaraldehyde is not a suitable product in the preparation of matrices suitable for culturing cells, since it makes the matrix poorly degradable and tends to block the proteins necessary for cell adhesion (Biopolymer methods in Tissue Engineering, Hollander P and Haltton PV ed., pg. 11, Humana Press, 2004). The glutaraldehyde remaining in the structure is very toxic for the cells and the cells seeded on the surface of the albumin-glutaraldehyde compounds are poorly bound and/or die. One possibility for placing cells therein is to mix albumin with the cells prior to the use of the cross-linking solution, however this is not possible either due to the aforementioned toxicity. There is in the state of the art a possibility of making this structure porous, such as the mixture with calcium carbonate type particles (WO 00/70018), however this forces treating the mixture with acid solutions to eliminate the particles, the residual glutaraldehyde remains and this product is toxic and not very recommendable as scaffold for the "in vitro" development of Tissue Engineering techniques.

Other albumin scaffolds have been described as porous either by the use of an innocuous porous building material such as PEG beads (WO2006/099137), NaCl (WO2004/108810), bicarbonate (US2002/059001) or by the preparation method itself (DE3517456)

The development of scaffolds or supports for Tissue Engineering from globular proteins such as albumin needs other systems which eliminate the toxicity of the cross-linking substance, provide a porous structure and if possible include therein motifs favoring the anchorage of the cells.

### Brief Description of the Invention

A first object of the present invention is a method for obtaining non-toxic three-dimensional structures to develop tissue engineering models from plasma globular proteins (namely albumin), cross-linked by a cross-linking agent (preferably glutaraldehyde). The protein-cross-linking agent mixture will be frozen and will subsequently be subjected to lyophilization. Once lyophilized, the resulting product must be hydrated so that it has resistance and elasticity. The hydration is performed by means of using ethanol in decreasing concentrations. Finally, the excess ethanol is washed in culture medium or balanced saline solution. A porous, flexible support which can be easily cut without breaking is thus generated.

A second object of the invention is the three-dimensional structure or scaffold obtainable by the aforementioned method.

The structure or scaffold thus designed can be used after its seeding with different cell types in a method for regenerating ex vivo the damaged tissue or organ. The cells adhere to this structure and are capable of growing and/or differentiating, starting the extracellular matrix protein synthesis.

Finally, the scaffold of the invention with the cells inside it can be transplanted to a living being, in which the immune response will cause a progressive reabsorption of the scaffold and the cells will produce the normal extracellular matrix of the tissue.

The final result will be the repair of a damaged organ or tissues by means of Tissue Engineering.

### Brief Description of the Drawings

Figure 1: the image depicts the support or scaffold of the invention observed in the scanning microscope (X500) made from 20% albumin (left) and a histological section thereof (right).
Figure 2: adhesion of human fibroblasts to a support or scaffold made directly with human plasma. At the right, expression of type I collagen (the most important extracellular protein of the dermis) by these fibroblasts.
Figure 3: preadipocytes extracted from rabbit subcutaneous fat , seeded on a structure made directly with rabbit serum. The cells were cultured for 45 days in an oven in adipogenic medium (left, oil red stain) and osteogenic medium (right, Von Kossa stain).
Figure 4: three-dimensional structure according to the invention observed with a scanning electron microscope (X200). At the right, support made with 5% albumin and at the left, with 20% albumin.
Figure 5: Image taken by a scanning microscope (X4000). Surface of the different supports. At the top and at the left, 20% albumin, at the right support directly made with serum. Bottom. Support made from plasma.
Figure 6: diagram of a prototype of the support of this invention inside an artificial dermis based on plasma and fibroblasts. The support provides the dermis based on fibrin and fibroblasts with a consistency facilitating the transplant thereof.
Figure 7: Structure of a three-layer skin: in the bottom part adipocytes differentiated on the scaffold of this invention, in the middle part fibroblasts in plasma gel and in the upper part keratinocytes.

### Detailed Description of the Invention

Firstly, the invention relates to a method for obtaining three-dimensional structures for tissue engineering comprising:
a) mixing a source of albumin and a cross-linking agent and introducing the mixture in a mold with the shape of the structure which is to be obtained
b) slowly and progressively freezing the solid structure obtained in a)
c) lyophilizing the structure of b)
d) progressive rehydrating the structure of c) by means of successive immersion in alcohols of decreasing strength.

In the context of the invention the source of albumin can be a purified albumin preparation or can be albumin directly obtained from the serum or plasma of the actual patient in whom the structure or scaffold with the cells will be implanted. The fact that the patient's own serum or plasma is used has the advantage that the immune implant rejection response is minimized. Furthermore, the use of plasma or serum of the patient compared to albumin preparations has the advantage that it provides more motifs of binding or anchorage to the cells which will subsequently be seeded in the structure of the invention, since it not only has the motifs typical of albumin but also of the rest of proteins present in blood.

The albumin concentration used for preparing the three-dimensional structures of the invention will depend on the application which will be given to it, i.e., on the type of tissue which is to be regenerated. Concentrations of between 1-50% of albumin can generally be used.

Any cross-linking agent causing the effect of denaturing and cross-linking the albumin molecules can be used as cross-linking agent although the use of aldehyde type agents such as formaldehyde or glutaraldehyde is preferred. The latter is especially preferred. The concentration of the cross-linking agent used in the mixture with the source of albumin can be at 0.1-9%, preferably at 0.5-7.5%.

The reaction of the albumin-cross-linking agent mixture in a mold with a predetermined shape allows the resulting structure to acquire the shape of the mold when it becomes solid. The shape of the structure can thus be adapted to the defect which is to be remedied or the damaged tissue which is to be regenerated.

After the slow and progressive freezing of the three-dimensional structure obtained, preferably at a rate of 1º C/min to a temperature of -70º C, a key step of the invention is carried out. This step means subjecting the solid structure obtained after the cross-linking and the freezing to a lyophilization. The lyophilization causes the cancellation of the toxic effect of the cross-linking agent but furthermore, produces a highly porous material, since the entire aqueous fraction of the scaffold is eliminated (while at the same time the cross-linking agent that is not bound to the globular protein is eliminated).

The product thus obtained is very friable and does not offer sufficient mechanical strength for its use. In order to improve these characteristics, this lyophilized product must be hydrated. This hydration must preferably be performed slowly and progressively to prevent breaking. The hydration will be performed by means of the treatment with alcohols in decreasing strength, preferably by means of the immersion in absolute alcohol, 96º, 90º, 80º, 70º and 50º alcohol. The structure obtained after the hydration will be washed in culture medium or in balanced salt solution to eliminate the alcohol remains that may still be present.

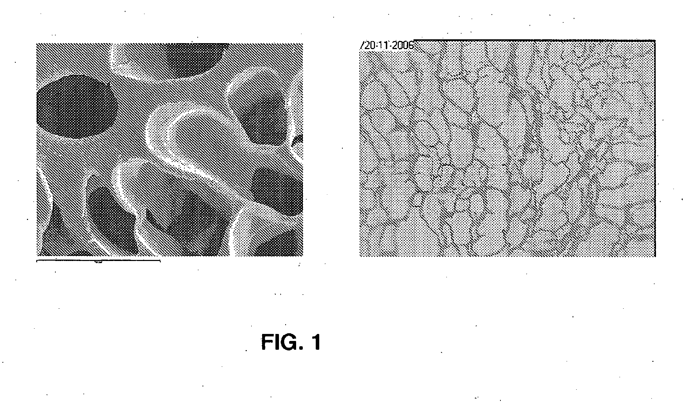

The final result of this method which is also object of the invention, is a three-dimensional porous, elastic, non-toxic structure (Figure 1) in which cultured cells which are capable of adhering to the scaffold of the invention can be seeded.

The following object of the present invention arises as a result of this capacity of the three-dimensional structure to adhere cells. This object is an ex vivo method for regenerating a tissue comprising:
a) seeding cells in the previously described three-dimensional structure;
b) incubating the cells in a culture medium inside an oven or bioreactor until the time in which the structure is to be implanted in the patient.

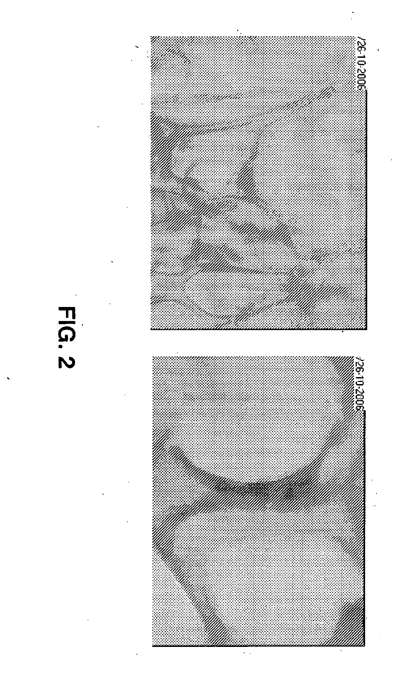

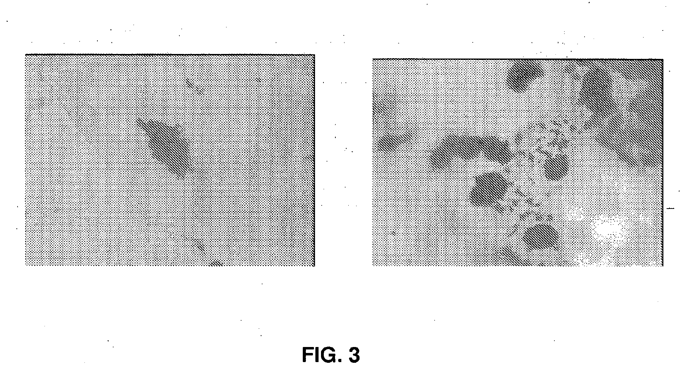

The three-dimensional structure or scaffold with these cells seeded inside it either by stirring, intermittent stirring or in a bioreactor, can be maintained "in vitro" in cell culture ovens or in bioreactors. During this period, the cells can continue growing, behave physiologically (Figure 2) and according to the growth or differentiation factors present in the culture medium, express the complete phenotype of the cell strain seeded or be differentiated towards cells of other types of tissues (Figure 3). This growth and/or differentiation occurs without observing a degradation of the structural part of the scaffold of the invention, even for periods of up to 6 months of "in vitro" culture. In a preferred embodiment of the invention the seeded and cultured and/or differentiated cells are osteoblasts, preadipocytes, chondrocytes or dermal fibroblasts.

Finally, the product of the invention thus manipulated which is also object of the invention can be transplanted to a living being, in which the structure generated by the cross-linking of albumin will be degraded by the immune system of the individual, the cells supplied will continue with the production of extracellular matrix which will be slowly substitute the initial protein structure. The result of this transplant will generate as a final product a novel tissue or organ capable of replacing the damaged part, the final objective of the Tissue Engineering processes.

### Embodiment of the Invention

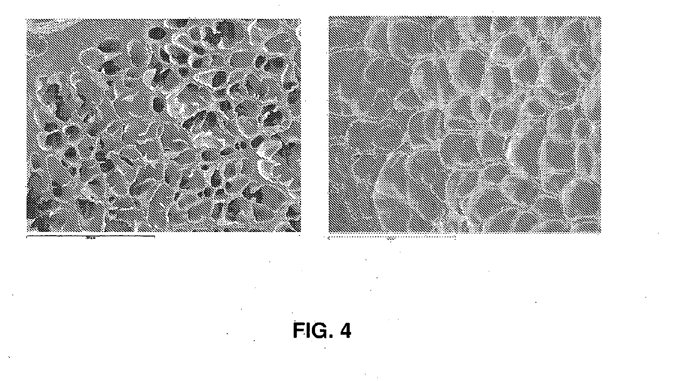

The basis of the product of the invention is the plasma globular proteins cross-linked with an aldehyde type substance. Albumin is the main plasma protein and is the structural basis of the product and can be used at different concentrations with different structural results (between 50 and 4%) (Figure 4). The cross-linking substance, for example the glutaraldehyde can also be used at different concentrations, between 0.5 and 7.5% with respect to the volume of albumin.

After the mixture, it is deposited in a mold and fast solidification occurs. The product is demolded and subjected to a slow and progressive freezing. Once the product is frozen it is subjected to lyophilization, when this ends, the product of this invention has a porous aspect but is extremely friable and breaks with a minimum force. To achieve a product which is usable as support or scaffold it must be hydrated. The hydration causes a drastic change in the physical characteristics of the product, rendering it elastic and resistant to handling. If this product is abruptly hydrated, part of the structure can break, it is therefore convenient to subject it to a progressive hydration. To that end, the product is introduced in absolute ethyl alcohol (between one and eight hours depending on the size of the structure), it is subsequently introduced in 96º, 90º and 80º alcohol for the same time period. After passing through the 80º alcohol the physical characteristics of the product change, the product is more elastic, the pores are more visible and it can be cut into fine sheets. The hydration of the product is continued, leaving it in 70º alcohol for 24 hours, 50º alcohol and, from here, in culture medium (DMEM, RPMI...) or balanced saline solutions. The saline solution is changed at least 3 times to eliminate all the alcohol remains present. The final product is an elastic sponge in which the pores can be clearly seen. This product can be stored in the culture medium for months without losing its functional capacity.

It has been previously mentioned that the albumin concentration for making the product could be very variable. When low albumin concentrations (4%) are used, the product is slightly less resistant than with 20% albumins. However, when low albumin concentrations are used the product is still stable and elastic. Human plasma contains between 3 and 5% albumin, this scaffold could therefore be made directly from human plasma. The direct use of plasma or serum in the production of the scaffold allows the possibility of manufacturing a three-dimensional structure starting directly from the blood of the patient in whom it will be subsequently implanted. To that end, by means of venipuncture, a small amount of blood would be extracted from the patient (between 10 and 100 ml) depending on the tissue to be reconstructed, in medium without anticoagulant (serum) or with it (plasma). It is centrifuged to remove the cellular component of the blood and mixed with the glutaraldehyde, it is placed in the mold and the product is left to solidify. After that moment, the slow freezing, the lyophilization and the progressive hydration is continued. The final result is a product which is apparently similar to the one prepared with albumin at low concentrations, a three-dimensional structure, in which mammalian cells can be seeded. This support has completely lost its toxicity. The supports/scaffolds generated from plasma or serum are clearly different from those generated directly from albumin concentrates, since other proteins normally present in the blood are also present in them. This difference also causes important changes in the function of the scaffold, which will be mentioned in the following paragraph.

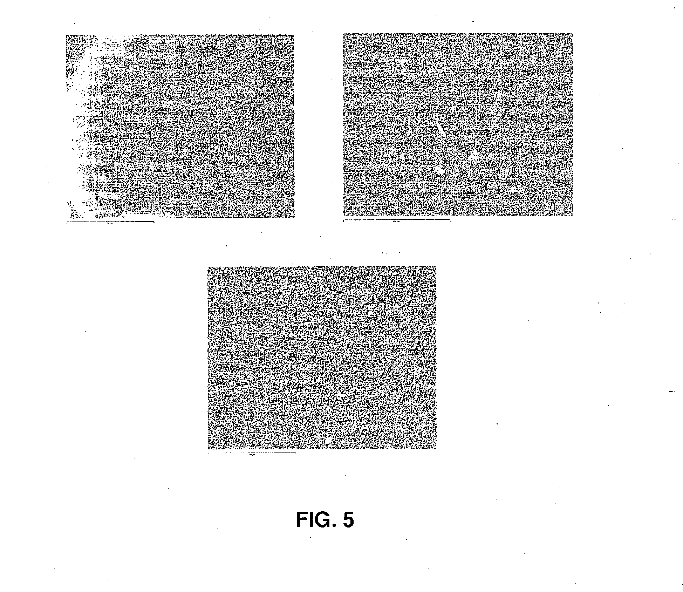

Once the three-dimensional structure is achieved, the cells are seeded. One of the greatest problems of most previously designed scaffolds is that they do not provide the signals necessary for facilitating the anchorage thereof to the support, since the cells-support interaction is a dynamic process in which the cells recognize a favorable surface and once the physical contact is initiated, the cells start synthesizing the specific extracellular matrix binding proteins. Previous studies performed with the scaffold of this invention show that the cells have a limited capacity to bind directly to albumin scaffolds, however, this capacity increases until becoming at least 10 times greater when scaffolds made directly with serum or preferably with plasma are used. The structural study performed by means of scanning electron microscopy shows that the surface of these structures is very different (Figure 5). These clear differences are due to the fact that there are many more proteins in serum and in plasma than in a purified albumin preparation and part of these proteins will be trapped inside the cross-linking occurring between albumin and the cross-linking agent.

This enables, depending on the strategy to be followed, using different scaffolds depending on whether a greater proliferation of the cells (preferably the cells grow better in adhesion) or a greater differentiation thereof is of interest.

Once the scaffold is achieved, it can be stored without losing its characteristics, leaving it in culture medium, or it can be used. To that end the cells typical of the tissue to be regenerated are seeded. Various strategies (stirring, intermittent stirring, bioreactor...) can be followed for the seeding.

The scaffold seeded with the cell component will then be in the oven or bioreactor until the time of the implant. This time period will be very variable according to the cell type used and the degree of differentiation required by the cells. During this period it can be observed how the cells are fixed to the scaffold and how they start producing the specific proteins of adult tissue. For example, if fibroblasts are seeded in a scaffold according to the invention and it is left in a typical growth medium (for example DMEM, 10% fetal bovine serum) it is observed how a few days after the seeding the fibroblasts synthesize type I collagen (Figure 2). If for example cells cultured from bone implants are seeded in this scaffold and these cells are left in osteogenesis medium it is observed how after a few days the cells deposit calcium salts on the scaffold, and express the alkaline phosphatase enzyme. If cultured chondrocytes are seeded and the scaffold is placed in chondral differentiation medium the production of type II collagen will be observed. When more undifferentiated cells are seeded, the periods necessary for the interval between the seeding and the scaffold can be longer. An example would be when mesenchymal stem cells obtained from a subcutaneous fat biopsy are seeded and left in osteogenic medium, a period greater than 15-20 days of "ex vivo" culture is necessary to see the expression of proteins typical of the bone. Despite these such extended periods the scaffold of this invention is not digested (or is minimally digested) and preserves the three-dimensional structure without alterations in "ex vivo" culture up to 6 months after the seeding.

Finally and once the structure and the desired cell differentiation are achieved "ex vivo", the scaffold of this invention can be transplanted.

After the transplant a scaffold always behaves as a foreign body and will generate an inflammatory response. This response must be moderate and cause a gradual and controlled degradation of the foreign material. The "in vivo" studies related to the scaffold of this invention show a very moderate degradation without observing a high inflammation in the area of the transplant. The integration of the new extracellular matrix produced by the cells seeded in the scaffold occurs while the structure of the original scaffold is degraded, a new tissue which can reproduce the functions of the originally damaged tissue being generated.

In summary, the scaffold of this invention allows a series of advantages which clearly differentiate it from what was previously known in the state of the art:
- It provides a three-dimensional structure for the cells, but also provides adhesion signals at the same time. This confers to the product an ideal composition for the development of Tissue Engineering models.
- It is a scaffold which allows the "in vitro" culture for long periods (up to 6 months), without losing the three-dimensional structure. This allows the development of "in vitro" differentiation models.
- It is a biological material widely used in clinical practice.
- The original product is very easily obtained (albumin concentrates) or total blood (venipuncture).
- The possibility of constructing a support for tissue engineering from small amounts of total blood offers the possibility of obtaining these structures starting from autologous biological products, i.e., obtained from the patient in which they are to be implanted himself.

The following examples describe the use of this scaffold in regenerative medicine, although they do not intend to be limiting with respect to the scope of the invention.

### Example 1: Preparation of a scaffold for repairing an arthrosis in a femoral diaphysis with 20 % albumin

The standard scaffold for pseudoarthrosis in a femoral diaphysis has an approximate dimension of 3 cm in diameter by 2 cm in height. For its preparation, the starting material is 10 ml of 20% human albumin of the type commonly used in clinical practice for its infusion. The albumin was mixed with 1 ml of 25% glutaraldehyde and immediately afterwards it was deposited in a mold with the aforementioned dimensions. The mixture was left to solidify at room temperature for 30-45 minutes and it was subsequently placed in an electric refrigerator at - 80ºC for 18-24 hours. Once frozen, the scaffold was demolded and, without thawing, it was introduced in the lyophilizer until the sample was completely lyophilized. Once this process had ended, the sample was placed in absolute ethyl alcohol for 2 hours. Then, the scaffold was passed to 96% ethanol and left for another 2 hours. The 96% ethanol was substituted with 80% ethanol and subsequently with 70% ethanol, in which it was left at room temperature for 24 hours. After this incubation, the scaffold was introduced for 2 hours in 50% ethanol, in sterile pure water and finally in an isotonic PBS type solution, Ham solution or even RPMI or DMEM type culture medium. It was washed several times with this solution to eliminate all the ethanol remains that may be in the product. After this incubation, a minimum sample of the scaffold was taken for bacteriological control and the scaffold was left in the saline solution until its use.

### Example 2: Preparation of a scaffold for repairing articular cartilage with plasma from the patient

Plasma from the patient himself was directly used for repairing an acute injury of the knee articular cartilage. Firstly, the chondral injury to be repaired was measured approximately by means of radiographic methods (nuclear magnetic resonance) or even better during the arthroscopic examination of the injured knee. At the same time, this arthroscopy allowed taking a minimum biopsy of the healthy articular cartilage for the culture and "ex vivo" expansion of these cells.

10 ml of blood were also extracted in EDTA at this moment, this blood was centrifuged, for example at 3000 rpm for 10 minutes. After the centrifugation, plasma was collected, 3 ml of this plasma were mixed with 300 µl of 25%glutaraldehyde and placed in a Petri dish of 35 mm in diameter, the mixture was left to solidify (30-60 minutes) and placed in an electric refrigerator at -80ºC until the following day. It was demolded without thawing and placed in a lyophilizer until the product was completely lyophilized. Afterwards, it was introduced in absolute ethanol for 2 hours and passed through 96%, 80% and 70% ethanol, in which it stayed for 18-24 hours.

It was subsequently placed for 2 hours in 50% ethanol and in PBS type saline solution or culture medium. Sample was taken for the bacteriological control of the scaffold thus prepared. It was washed at least 3 times with the saline solution to eliminate the ethanol remains. In this phase, the size of the scaffold was adjusted to that of the cartilaginous injury, cutting the latter with a scalpel for example. The scaffold was ready for being seeded with cells.

### Example 3: Regeneration of maxillary bone for dental implants

10 ml of blood were extracted from a patient in need of dental implants and who did not having enough bone mass and a process similar to that of Example 2, but using a mold very similar to the defect which had to be regenerated, was carried out. At the same time, some small spongy fragments were extracted from the maxilla. After freezing, lyophilizing and hydrating the material, the scaffold was set aside for seeding cells and implanting. The bone fragments of the patient were seeded on a cell culture flask by the explant technique, and there was a wait until the viable cells present therein grew. After the growth, the cells were subcultured according to the different described techniques. Once a sufficient cell mass of bone cells was achieved, they were seeded on the scaffold, left for a time period in the oven so that the cells could bind and were transplanted to the area to be reconstructed. The scaffold was slowly degraded and the cells contained therein produced the adult matrix of the bone.

### Example 4: Regeneration of maxillary bone for dental implants

Another strategy similar to that of Example 3 was followed with another patient. Instead of extracting a bone fragment, a small subcutaneous fat biopsy was used as cell source. The fat was digested in collagenase and the cells were seeded in a cell culture flask in growth medium (DMEM, 10% fetal bovine serum). After a critical mass was achieved, the cultured preadipocytes were seeded on the scaffold and left in bone differentiation medium, until signs of differentiation towards osteoblasts were seen by the follow-up. The scaffold and the cells contained therein were subsequently transplanted to the maxillary defect to be regenerated.

### Example 5: Regeneration of an earlobe deformity

An earlobe deformity was repaired by means of using a scaffold of serum of the patient. The materials and methods are substantially those used in Example 2 although the scaffold was hardened on a mold which reproduced the structure of the ear cartilage. At the same time a small healthy sample of the auricular cartilage of the patient was taken. On the one hand, the scaffold was frozen, lyophilized and hydrated according to the methodology described in Example 2. On the other hand, the cartilage was digested subjecting it to proteolytic enzymes and the obtained chondrocytes were cultured until obtaining a cell mass sufficient to be seeded on the scaffold. After the seeding, the chondrocytes underwent a redifferentiation process by means of culturing in an oven (25-45 days) and were finally transplanted to the injured area.

This same strategy could be followed for repairing an articular cartilage, changing the origin of the source of chondrocytes.

### Example 6: Regeneration of subcutaneous fatty tissue

Blood was taken from the patient, the scaffold was made as described in Example 2 and seeded with preadipocytes cultured from a fat tissue biopsy of the patient. Once seeded on the scaffold, the later was left culturing in the oven in adipose differentiation medium until the seeded cells started accumulating triglycerides therein. It was subsequently transplanted to the region to be reconstructed.

This same strategy could be followed after mastectomies due to breast neoplasias.

### Example 7: Bone reconstruction

A scaffold for reconstructing bone injuries can be developed as in Example 1. However, some bone reconstructions can require a higher consistency of the scaffold than that based on plasma or serum, therefore a percentage of human albumin would be added to the plasma or serum of the patient to reinforce the structure, maintaining the cell anchorage properties. Finally this scaffold could be seeded with cells of the patient extracted from a bone biopsy or as in Example 4 from subcutaneous fat.

### Example 8. Dermal regeneration after a burn

The basis of this treatment consisted of the production of sheets with a small thickness of the scaffold of this invention, seeded from dermal fibroblasts of the patient himself and transplanted on the site of the injury. In this mode, the fibroblasts could be from another healthy patient given that the fibroblasts are cells with low capacity to generate immunological rejection. This prototype of scaffold in the form of a sheet plus dermal fibroblasts can be associated to semipermeable silicone type membranes providing a barrier effect and protecting the wound and the implant from possible infections.

This type of strategy could be used in other skin injuries (ulcers, diabetic foot surgical amputations).

### Example 9: Reinforcement structure

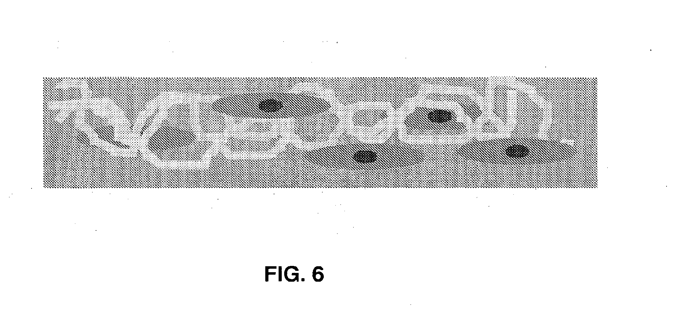

The scaffold of this invention can also be used as an internal reinforcement structure of other materials already used in tissue engineering. One example of this application is the association of a sheet of the prototype herein described to a plasma sheet containing living fibroblasts. In this example human plasma containing fibroblasts are seeded on a sheet of the scaffold of the invention, calcium chloride is added to coagulate the plasma and the scaffold is located inside the plasma, serving as an internal framework and facilitating the transplant of this artificial dermis. Also, if keratinocytes are seeded on this artificial dermis an artificial skin with an internal framework providing rigidity and facilitating the transplant is achieved. Figure 6 schematizes this prototype.

### Example 10: Artificial skin prototype

This prototype can be associated with an artificial skin model defined in Example 9 to generate a cultured three-layer skin incorporating subcutaneous fat.

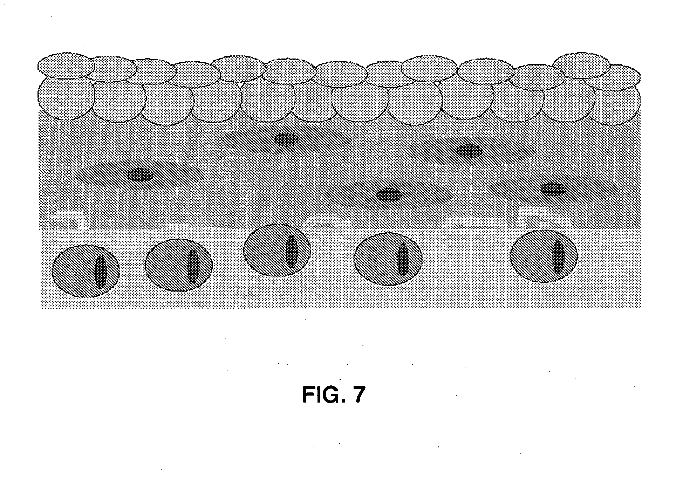

To that end, cells are obtained from a small fat biopsy, which cells are cultured in an expansion medium (DMEM, 10% bovine serum). When a sufficient number of cells is reached, the cells are seeded in the scaffold of this invention and cultured in a medium of differentiation towards adipocyte. When the cells present signs of fat differentiation (Figure 3, left), dermal fibroblasts, plasma are added to the scaffold and recalcified to cause the coagulation. The epidermal keratinocytes are seeded on its surface and cultured until becoming confluent. The model which would be obtained would be a bottom part with fat cells bound to the scaffold of this invention, an immediately upper layer of plasma with fibroblasts similar to the human dermis being bound to it and in the upper part a epithelial layer (Figure 7), i.e. a human skin with 3 layers much more similar to the natural one than the one generated with different strategies.

## Claims

1. Method for obtaining three-dimensional structures for tissue engineering comprising:
a) mixing a source of albumin and a cross-linking agent and introducing the mixture in a mold with the shape of the structure which is to be obtained
b) slowly and progressively freezing the solid structure obtained in a)
c) lyophilizing the structure of b)
d) progressive rehydrating the structure of c) by means of successive immersion in alcohols of decreasing strength.

2. Method according to claim 1, wherein the source of albumin is selected from a purified albumin preparation, serum or plasma

3. Method according to claim 1, wherein the cross-linking agent is an aldehyde.

4. Method according to claim 3, wherein the aldehyde is glutaraldehyde.

5. Method according to claim 1, wherein the source of albumin has an albumin concentration between 1% and 50%.

6. Method according to claim 5, wherein the source of albumin has a concentration of 3-5%.

7. Method according to claim 1, wherein the cross-linking agent has a concentration between 0.1 and 9%.

8. Method according to claim 7, wherein the cross-linking agent has a concentration of 0.5-7.5%.

9. Method according to claim 1, wherein the freezing is performed at a rate of -1° C/min to a temperature of -70° C.

10. Method according to claim 1, wherein the progressive rehydration is carried out by successive immersion in absolute alcohol, 96°, 90°, 80°, 70°, 50° alcohol and finally in culture medium or balanced saline solutions.

## Patentansprüche

1. Verfahren zur Gewinnung von dreidimensionalen Strukturen für das Tissue Engineering, umfassend:
a) Mischen einer Quelle für Albumin und eines Vernetzungsmittels und Einführen des Gemischs in eine Form mit der Form der zu erhaltenden Struktur;
b) langsames und fortschreitendes Gefrierenlassen der in a) erhaltenen festen Struktur;
c) Lyophilisieren der Struktur von b);
d) fortschreitendes Rehydratisieren der Struktur von c) mittels sukzessiven Eintauchens in Alkohole abnehmender Stärke.

2. Verfahren gemäß Anspruch 1, wobei die Quelle für Albumin aus einem gereinigten Albuminpräparat, Serum oder Plasma ausgewählt ist.

3. Verfahren gemäß Anspruch 1, wobei das Vernetzungsmittel ein Aldehyd ist.

4. Verfahren gemäß Anspruch 3, wobei es sich bei dem Aldehyd um Glutaraldehyd handelt.

5. Verfahren gemäß Anspruch 1, wobei die Quelle für Albumin eine Albuminkonzentration zwischen 1% und 50% aufweist.

6. Verfahren gemäß Anspruch 5, wobei die Quelle für Albumin eine Konzentration von 3-5% aufweist.

7. Verfahren gemäß Anspruch 1, wobei das Vernetzungsmittel eine Konzentration zwischen 0,1 und 9% aufweist.

8. Verfahren gemäß Anspruch 7, wobei das Vernetzungsmittel eine Konzentration von 0,5-7,5% aufweist.

9. Verfahren gemäß Anspruch 1, wobei das Gefrierenlassen mit einer Geschwindigkeit von -1°C/min auf eine Temperatur von -70°C durchgeführt wird.

10. Verfahren gemäß Anspruch 1, wobei das fortschreitende Rehydratisieren durch sukzessives Eintauchen in absoluten Alkohol, Alkohol von 96%, 90%, 80%, 70%, 50% und schließlich in Kulturmedium oder isotonische Kochsalzlösungen durchgeführt wird.

## Revendications

1. Procédé d'obtention de structures tridimensionnelles destinées au génie tissulaire, comprenant :
a) le mélange d'une source d'albumine et d'un agent de réticulation et l'introduction du mélange dans un moule ayant la forme de la structure qui est à obtenir,
b) la congélation lente et progressive de la structure solide obtenue en a),
c) la lyophilisation de la structure de b),
d) la réhydratation progressive de la structure de c) par immersion successive dans des alcools de concentration décroissante.

2. Procédé selon la revendication 1, dans lequel la source d'albumine est choisie parmi une préparation d'albumine purifiée, le sérum ou le plasma.

3. Procédé selon la revendication 1, dans lequel l'agent de réticulation est un aldéhyde.

4. Procédé selon la revendication 3, dans lequel l'aldéhyde est le glutaraldéhyde.

5. Procédé selon la revendication 1, dans lequel la source d'albumine présente une concentration en albumine située entre 1 % et 50 %.

6. Procédé selon la revendication 5, dans lequel la source d'albumine présente une concentration de 3 % à 5 %.

7. Procédé selon la revendication 1, dans lequel l'agent de réticulation présente une concentration située entre 0,1 % et 9 %.

8. Procédé selon la revendication 7, dans lequel l'agent de réticulation présente une concentration de 0,5 % à 7,5 %.

9. Procédé selon la revendication 1, dans lequel la congélation est réalisée à une vitesse de - 1 °C/minute jusqu'à une température de -70 °C.

10. Procédé selon la revendication 1, dans lequel la réhydratation progressive est réalisée par immersion successive dans de l'alcool absolu à 96°, 90°, 80°, 70°, 50° et finalement dans un milieu de culture ou dans des solutions physiologiques salées équilibrées.
